# EUROPÄISCHE PATENTANMELDUNG

(11) **EP 1 810 747 A1**
(43) Veröffentlichungstag der Anmeldung: **25.07.2007**
(21) Anmeldenummer: 05749500.4
(22) Anmeldetag: 23.05.2005
(51) Int. Cl.: B01J 19/10

(54) **KAVITATIONSREAKTOR**

(30) Priorität: 16.06.2004 RU 2004118093
(71) Anmelder: Obschestvo S Ogranichennoi Otvetstvennostyu "Astor-s", Vologda, 160019 (RU); Shestakov, Sergei Dmitrievich, Vologda 160031 (RU)
(72) Erfinder: SHESTAKOV, Sergei Dmitrievich, Vologda, 160031 (RU)
(74) Vertreter: Jeck, Anton
(86) Internationale Anmeldenummer: PCT/RU2005/000280
(87) Internationale Veröffentlichungsnummer: WO 2005/123245

(57) **Zusammenfassung**

Die Erfindung bezieht sich auf einen Kavitationsreaktor zur Behandlung von Flüssigkeiten mit einer durch Wandungen eines Gehäuses begrenzten und mit einer zu behandelnden Flüssigkeit füllbaren Kammer, mindestens einer Schallwelle erzeugenden Schallquelle sowie mindestens einer Reflexionswand.

Der Kavitationsreaktor umfasst einen Festkörperresonator, der eine Resonanzfrequenz aufweist, die der Frequenz der Schallquelle entspricht. Diejenige Wandung des Resonators ist als die Reflexionswand ausgebildet, deren Oberfläche der Schallquelle zugewandt ist.

## Beschreibung

Die Erfindung betrifft eine Vorrichtung zur Behandlung von Flüssigkeiten durch mittels Schallwellen erzeugter Kavitation.

Eine Behandlung von Flüssigkeiten durch mittels Schallwellen erzeugter Kavitation kann beispielsweise eine Zerstörung und/oder Abtrennung und/oder Teilung verschiedener in einer Flüssigkeit enthaltener Substanzen, wie etwa die Abtötung von Mikroorganismen, die beispielsweise in Form von Schwebepartikeln in der Flüssigkeit vorkommen können, und/oder eine Dissoziation der Flüssigkeitsmoleküle zum Ziel haben. Hierzu vorgesehene Vorrichtungen zur Behandlung von Flüssigkeiten durch mittels Schallwellen erzeugter Kavitation werden auch als Kavitationsreaktoren bezeichnet. Der Kavitationsbehandlung in einem Kavitationsreaktor können beliebige Flüssigkeiten, wie etwa Emulsionen, Suspensionen, kolloidale bzw. echte Lösungen, sowie Wasser und andere Flüssigkeiten unterzogen werden. Die Kavitationsbehandlung einer Flüssigkeit kann beispielsweise zur Erhöhung der Dispersität und Homogenität der vorhandenen Phasen, zur Verstärkung und/oder Beschleunigung von in der Flüssigkeit ablaufenden chemischen Reaktionen, wie etwa der Synthese und/oder der Analyse und/oder der Bildung neuer Verbindungen nachfolgender Reaktionen, sowie zur Bakteriolyse und Bakteriostase dienen. Der Vorgang der Energieübertragung in einer Flüssigkeit durch Kavitationseinwirkung hat einen "epithermischen" Charakter, welcher der hochenergetischen Chemie eigene Prozesse sicherstellt, bei denen Wasser kurzzeitig aus dem thermodynamischen Gleichgewicht gebracht wird. Der Vorgang der Kavitationseinwirkung erlaubt eine bestimmte Menge Energie in der Flüssigkeit fast ohne Aufheizung zu akkumulieren und nachher diese Energie bei Rückkehr zum Gleichgewichtszustand als Hydratationswärme abzugeben.

Aus US 3519251 ist ein Kavitationsreaktor bekannt, bei dem eine Oberfläche eines Ultraschallgenerators in einer Ebene mit einer den Innenraum des Kavitationsreaktors begrenzenden Reflexionswand angeordnet ist. Die Oberfläche strahlt Ultraschallwellen in einen Innenraum des Kavitationsreaktors ab. Das heißt, die stirnseitige, elastische Oberfläche des Ultraschallgenerators, welche durch mechanische Schwingungsanregung Ultraschallwellen in den Innenraum abstrahlt, stimmt mit einer den Innenraum des Kavitationsreaktors einseitig begrenzenden ersten Reflexionswand überein. Eine zweite Reflexionswand, die sich in Ausbreitungsrichtung der Ultraschallwellen gegenüber der ersten Reflexionswand und gegenüber dem Ultraschallgenerator befindet, ist fest mit einem Gehäuse des Kavitationsreaktors verbunden.

Aus Knepp R., Daily G., Chemmit F., "Kavitacionnoj", Verlag Mir, Moskau, 1974, 348 Seiten, ist bekannt, dass die durch die Ultraschallwellen in die Flüssigkeit eingebrachte Kavitationsenergie, welche die gewünschten Effekte in der in den Kavitationsreaktor eingebrachten Flüssigkeit verursacht, auch eine erosionsartige Zersetzung bzw. Schädigung, oder gar Zerstörung des Kavitationsreaktors selbst verursacht. Demnach können durch die Kavitationsenergie auch Werkstoffe, aus denen einzelne Konstruktionselemente des Kavitationsreaktors hergestellt sind, Schädigungen erfahren oder gar zerstört werden. Die durch die Erosion aus den Konstruktionselementen freigesetzten Teilchen gelangen dabei in die im Kavitationsreaktor behandelte Flüssigkeit und können dadurch irreversibel die physischen und chemischen Eigenschaften der Flüssigkeit ändern. Insbesondere bei der Behandlung pharmazeutischer oder medizinischer Produkte oder Präparate, bei der Behandlung von Nahrungsmitteln sowie bei der Behandlung von Ausgangssubstanzen für Medikamente oder Nahrungsmittel ist dies nicht zulässig. Bei dem bekannten Kavitationsreaktor ist das gesamte, den Innenraum begrenzende Gehäuse der Erosion ausgesetzt. Die Erosion findet dabei zum einen an den Stellen am Innenumfang des Innenraums statt, welche an das durch den Betrieb des Kavitationsreaktors in der Flüssigkeit erzeugte Kavitationsgebiet angrenzen. Zum anderen findet Erosion auch an Stellen des Gehäuses statt, welche selbst zu Schwingungen angeregt, unmittelbar Kavitation in der Flüssigkeit erzeugen. Dieser Effekt wird dadurch verursacht, dass die mit dem Gehäuse fest verbundene, dem Ultraschallgenerator gegenüberliegende zweite Reflexionswand die auf sie treffenden Ultraschallwellen nicht vollständig reflektieren kann. Hierdurch werden die Ultraschallwellen teilweise in die zweite Reflexionswand eingekoppelt und von dort in das Gehäuse weitergeleitet. Dabei findet eine Modulation statt. Das Gehäuse, angeregt durch die eingekoppelten und modulierten Ultraschallwellen, schwingt dadurch selbst, wodurch an der Grenze zwischen Flüssigkeit und schwingenden Gehäusewandungen Kavitation entsteht. Der durch Kavitation verursachten Erosion werden nach Schestakov S.D., "Grundlagen der Technologie der Kavitationsdesintegration" EBA-Press, Moskau 2001, 173 Seiten, beim bekannten Kavitationsreaktor infolge der Ungleichheit ihrer Schwingungsamplituden auch die Oberfläche des Ultraschallgenerators sowie die den Ultraschallgenerator umgebende Reflexionswand ausgesetzt.

Aus US 4618263 ist ein Kavitationsreaktor mit einem einen Innenraum begrenzenden Gehäuse bekannt. In dem Innenraum befinden sich die zu behandelnde Flüssigkeit, eine Ultraschallquelle zur Kavitationserzeugung sowie eine Reflexionswand. Zwischen der Reflexionswand und dem Gehäuse ist eine Schicht angeordnet, die denjenigen Teil der Ultraschallwellen absorbiert, der nicht in die zu behandelnde Flüssigkeit reflektiert wurde. In einem solchen Kavitationsreaktor wird auf eine feste, mechanisch starre Verbindung zwischen Reflexionswand und Gehäuse verzichtet und die Verbindung stattdessen mittels der Schicht hergestellt. Hierdurch kann ein Teil der durch die Ultraschallwellen auf die Reflexionswand übertragenen Energie in der zwischen Reflexionswand und Gehäuse angeordneten Schicht abgebaut werden. Durch Dissipation wird in der Schicht ein Teil der in die Reflexionswand eingekoppelten Energie in Wärme umgewandelt, so dass nur noch ein Teil zum Gehäuse weitergeleitet wird. Hierdurch wird die durch Schwingungen der Reflexionswand und des Gehäuses entstehende Kavitation verringert. Bei Betrieb dieses Kavitationsreaktors werden jedoch die den Innenraum begrenzenden Wandungen durch stationäre Kavitationsgebiete einer stärkeren Kavitationseinwirkung ausgesetzt, als bei dem zuvor beschriebenen ersten Kavitationsreaktor. Die durch die stationären Kavitationsgebiete erzeugte Kavitation in der Nähe der Gehäuseoberfläche verursacht dabei nach wie vor eine Erosion der Werkstoffe der Konstruktionselemente des Gehäuses. Die durch die Erosion freigesetzten Teilchen gelangen dabei ebenso in die zu behandelnde Flüssigkeit, wodurch auch der zweite Kavitationsreaktor nicht zur Behandlung von Medikamenten oder Nahrungsmitteln geeignet ist.

Aus Schestakov S.D., "K theorie Kavitationnovo Reatora 2", Sessii rossijskogo Akusticeskogo Obschestva, M.: GEOS, Band 1, 2003 Seiten 31-35, ist ein weiterer Kavitationsreaktor zur Behandlung von Flüssigkeiten bekannt. Der Kavitationsreaktor weist eine Kammer mit einem durch Innenwandungen eines Gehäuses, durch eine Oberfläche eines Ultraschallgenerators sowie durch eine Reflexionswand begrenzten Innenraum auf. Auch bei diesem Kavitationsreaktor ist zwischen Reflexionswand und Gehäuse eine Schicht aus einem Energie absorbierenden Material angeordnet. Die Parameter des Kavitationsreaktors sind die Schicht des verwendeten Materials, die Größe der Schwingungsamplituden des Ultraschallgenerators, dessen Oberfläche und die Reflexionswände. Zur Anpassung wird je nach verwendetem Material der akustische Widerstand der Schicht verändert. Durch die Anpassung kann ein Ausgleich der Schwingungsamplituden der Oberfläche des Ultraschallgenerators und der Reflexionswand erzielt werden.

Reflexionswand erzielt werden. Hierdurch kann die Entstehung von Kavitationsgebieten an den die Kammer begrenzenden Wandungen des Kavitationsreaktors vermieden werden, wodurch deren Zerstörung durch Erosion verhindert wird. Der Ausgleich der Schwingungsamplituden des Ultraschallgenerators und der Reflexionswände wird bei dem Kavitationsreaktor durch Erzeugung eines Zustandes erreicht, der energetisch gesehen dem Zustand einer laufenden Welle ähnlich ist. Dieser Zustand entspricht einer Situation, bei der die auf die Reflexionswand treffenden Schallwellen ohne in den Kavitationsreaktor zurück geworfen zu werden, durch die Reflexionswand hindurch gehen würden. Das heißt, die durch die Schicht aus Energie absorbierendem Material am Gehäuse des Kavitationsreaktors befestigte, die Kammer begrenzende Wand ist nur bedingt als Reflexionswand geeignet. Der Teil der Energie, die von der Schallwelle über die Wand in die Schicht übertragen wird, wird durch innere Reibung im Energie absorbierenden Material der Schicht vernichtet. Dabei wird nur ein Teil der Energie, die von den vom Ultraschallgenerator erzeugten Schallwellen in die zu behandelnde Flüssigkeit übertragen wird, zur Erzeugung der Kavitation genutzt. Das Auftreten von Erosion an den die Kammer begrenzenden Wandungen des Kavitationsreaktors wird demnach nur durch einen Verlust eines Teils der Energie der in die Flüssigkeit übertragenen Schallwellen verursacht. Hierdurch wird zur Behandlung einer Flüssigkeit mehr Energie benötigt, um diese Verluste zu ersetzen.

Aus Gorelik G.S., "Kolebanija i volny-M.:", F-ML, Moskau, 1959, 572 Seiten, ist bekannt, dass nicht nur bei laufenden Wellen die Schwingungsamplituden benachbarter Schwingungsbäuche im Wesentlichen identisch sind, sondern dass dies auch bei stehenden Wellen zutrifft. im letzteren Fall würde nach Schestakov S.D., "Grundlagen der Technologie der Kavitationsdesintegration" EBA-Press, Moskau 2001, 173 Seiten kein Kavitationsgebiet an der Oberfläche des Ultraschallgenerators entstehen, wodurch hier auch keine Erosion auftreten würde. Bei stehenden elastischen Wellen findet ein Energieaustausch darüber hinaus nur zwischen den benachbarten Halbwellenvolumina statt. Dadurch werden die Energieverluste auf ein Minimum reduziert. Befinden sich diese Halbwellenvolumina innerhalb des Kavitationsreaktors in der zu behandelnden Flüssigkeit, so wird die gesamte Energie der Schallwelle in der Flüssigkeit verteilt. Dieser Vorteil kann ausgenutzt werden, da bei den bekannten Ultraschallgeneratoren der Effekt der akustischen und nicht der hydrodynamischen Kavitation zu Tragen kommt. Sämtliche für Ultraschallgeneratoren der bekannten Kavitationsreaktoren verwendeten Ultraschallquellen sind hierbei als Resonatoren ausgeführt, wodurch es prinzipiell möglich sein sollte, einen gesamten Kavitationsreaktor in Resonanz zu betreiben.

Es ist bekannt, dass nicht nur im Modus der laufenden Welle die Versetzungsamplituden der im nachbarlichen Amplitudebauch nach der Größe [6] praktisch gleich sind. Dasselbe ist im Modus der stehenden Welle zu sehen. In diesem Fall wird der Kavitationsbereich an der Oberfläche des Strahlers im Reaktor auch [3] fehlen und seine erosive Zerstörung wird ausgeschlossen. Dabei werden die Energieverluste auf Minimum reduziert, da es bekannt ist, dass in der stehenden elastischen Welle der Austausch von Energien nur zwischen den benachbarten Halbwellenvolumen [6] erfolgt. Wenn sich diese Volumen innerhalb des Reaktors in der zu bearbeitenden Flüssigkeit befinden, so wird die ganze Energie der Welle darin zerstreut. Diesen Vorteil ausnutzen erlaubt die Tatsache, dass alle bekannten Quellen der Ultraschallwelle, die als Energiequellen in den Kavitationsreaktoren verwendet werden, die aufgrund der Erscheinung der akustischen (nicht hydrodynamischen) Kavitation funktionieren, die Resonanzschwingungssysteme - Resonatoren sind. Wenn im Kavitationsreaktor als Quelle der Schallwelle ein Resonator vorhanden ist, kann ein Resonanzzustand des gesamten Kavitationsreaktors verwirklicht werden.

Eine Aufgabe der Erfindung ist es demnach, einen Kavitationsreaktor zur Behandlung von Flüssigkeiten vorzuschlagen, der eine Verringerung der Energieverluste einer Schallwelle ermöglicht, welche in einer in den Kavitationsreaktor eingebrachten Flüssigkeit Kavitation erzeugt. Gleichzeitig soll eine Senkung der durch Kavitation verursachten Erosion an der Oberfläche eines die Schallwelle erzeugenden Ultraschallgenerators erreicht werden.

Die gestellte Aufgabe wird durch die Merkmale des Anspruchs 1 gelöst. Der erfindungsgemäße Kavitationsreaktor zur Behandlung von Flüssigkeiten weist demnach eine durch Wandungen eines Kavitationsreaktorgehäuses begrenzte, mit einer zu behandelnden Flüssigkeit füllbare Kammer, mindestens eine die Schallwelle erzeugende Schallquelle sowie mindestens eine Reflexionswand auf. Erfindungsgemäß umfasst der Kavitationsreaktor einen Festkörperresonator, der eine Resonanzfrequenz aufweist, die der Frequenz der Schallquelle entspricht. Dabei ist die der Schallquelle zugewandte Oberfläche des Festkörperresonators als die Reflexionswand ausgebildet.

Gemäß einer bevorzugten Ausgestaltung der Erfindung sind die einander zugewandten Oberflächen der Schallquelle und des Resonators im Schwingungsbauch der zur Behandlung der in die Kammer einbringbaren Flüssigkeit eingesetzten Schallwelle angeordnet.

Gemäß einer anderen bevorzugten Ausgestaltung der Erfindung sind die Schallquelle und der Resonator derart miteinander verbunden, dass sie ein einheitliches Festkörperschwingungssystem bilden.

Bei dem erfindungsgemäßen Kavitationsreaktor bildet sich in der Flüssigkeit eine stehende Welle. Deren Druckamplitude, also die in der Flüssigkeit verteilte Energie, ist im Vergleich zu einem eine laufende Welle ausbildenden Kavitationsreaktor nach dem Stand der Technik bei Verwendung einer Schallquelle gleicher Leistung viel größer. Darüber hinaus sind beim erfindungsgemäßen Kavitationsreaktor die Schwingungsamplituden an der Oberfläche der Schallquelle und an der gegenüber liegenden Reflexionswand in dem Fall, dass die Oberflächen der Schallquelle und der Reflexionswand gleich groß sind, ebenso gleich groß. Sind die Oberflächen der Schallquelle und der Reflexionswand unterschiedlich groß, so sind nach Schestakov S.D., "Grundlagen der Technologie der Kavitationsdesintegration", EBA-Press, Moskau, 2001, 173 Seiten, die Schwingungsamplituden umgekehrt proportional dem Größenverhältnis der Oberflächen. Hierdurch wird die Entstehung eines Kavitationsgebiets an der Oberfläche der Schallquelle und an der Oberfläche der Reflexionswand verhindert, wodurch es auch nicht zu Erosion kommt.

Ausführungsbeispiele der Erfindung werden nachfolgend anhand der Zeichnungen erläutert. Dabei zeigen:
- Fig. 1:: eine schematische Darstellung eines Kavitationsreaktors mit rundem Querschnitt für den Fall, dass die Reflexionswand eine Wandung eines Resonators ist, der ein selbständiges Schwingungssystem für eine stehende Welle bildet, sowie
- Fig. 2:: eine schematische Darstellung eines Details eines Kavitationsreaktors mit rundem Querschnitt für den Fall, dass die Reflexionswand eine Wandung eines Resonators ist, der einen Teil des Schwingungssystems der Schallquelle bildet.

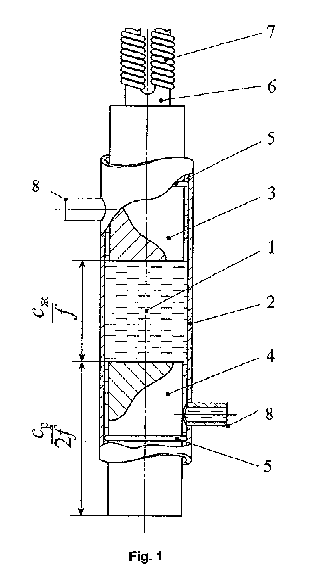

Ein in Fig. 1 dargestellter erfindungsgemäßer Kavitationsreaktor zur Behandlung von Flüssigkeiten umfasst eine mit der zu behandelnden Flüssigkeit gefüllte, beispielsweise zylinderförmige Kammer 1. Die den Innenraum der Kammer 1 begrenzenden Wandungen bestehend aus Innenflächen eines Gehäuses 2, den dem Innenraum der Kammer 1 zugewandten Oberfläche eine Ultraschallquelle 3 sowie der Oberfläche einer Reflexionswand. Die Ultraschallquelle 3 erzeugt Ultraschallwellen mit einer Frequenz f. Als Reflexionswand dient eine der Ultraschallquelle 3 zugewandte Oberfläche eines Festkörperresonators 4 mit einer Resonanzfrequenz, die der Frequenz f der Ultraschallwellen entspricht. Die Ultraschallquelle 3 und der Resonator 4 sind als Zylinder ausgebildet und sind in den Schwingungsknoten mit den Vorrichtungen 5 fixiert. Die Vorrichtungen 5 können beispielsweise Klebeverbindungen oder ein- oder mehrlagige Schichten aus geeigneten Materialien sein.

Wenn die Schallgeschwindigkeit im Material, aus dem der Resonator 4 hergestellt ist, gleich Cp, und in der zu behandelnden Flüssigkeit gleich C_{∗/zh} ist, dann beträgt die Zylinderhöhe des Resonators vorzugsweise Cₚ/2f. Vorzugsweise beträgt der Abstand zwischen der Oberfläche der Ultraschallquelle 3 und der Reflexionswand c_{∗/zh} /f, da deren Oberflächen vorzugsweise jeweils in einem Schwingungsbauch der stehenden Welle mit der Schwingungsfrequenz f der Ultraschallquelle 3 liegen. Allgemein soll dieser Abstand vorzugsweise durch C_{∗/zh/}2f teilbar sein.

Zur Erzeugung der Ultraschallwelle dient vorzugsweise ein Ultraschallgenerator. Der Ultraschallgenerator ist als ein in einem Schwingungsbauch angeordneter elektroakustischer Magnetostriktionsumwandler 6 mit einer Erregerwickelung 7 ausgeführt. Zur Durchleitung der zu behandelnden Flüssigkeit durch den Kavitationsreaktor dienen die Stutzen 8.

Der in Fig. 1 dargestellte Kavitationsreaktor arbeitet wie folgt. In der zu behandelnden Flüssigkeit, die durch die Kammer 1 des Kavitationsreaktors durch den Stutzen 8 durchgeleitet wird, verbreitet sich ausgehend von der der Kammer 1 zugewandten Oberfläche der Ultraschallquelle 3 eine vom elektroakustischen Wandler 6 erzeugte Schallwelle. Die Strömungsrichtung der Flüssigkeit hat dabei keinerlei Auswirkung auf das erzielbare technische Ergebnis. Im Schwingungssystem, bestehend aus dem elektroakustischen Wandler 6, der Ultraschallquelle 3, dem Volumen der Flüssigkeit in der Kammer 1 zwischen den Oberflächen des Strahlers und des Resonators 4 sowie des Resonators 4 selbst, entstehen Resonanzschwingungen. Hierdurch wird ein Zustand erzeugt, bei dem sich eine stehende Schallwelle in der Flüssigkeit ausbildet, welche die zur Behandlung der Flüssigkeit erwünschte Kavitation hervorruft.

Ein Teil der Energie der Schallwelle wird dabei durch innere Reibung in der Flüssigkeit und im Material des Resonators 4 in Wärme umgewandelt und geht damit verloren. Aus Bergmann, L. "Ultrazwuk i evo Primenije v Nauke i technike.-M:", IIL, Moskau, 1956, 726 Seiten, illustriert, ist bekannt, dass die Verluste durch innere Reibung in Festkörpern kleiner sind als in Flüssigkeiten. Außerdem wird im Gegensatz zum Stand der Technik der Großteil der Energie der Schallwelle, der durch die Flüssigkeit hindurch die Reflexionswand erreicht hat, in die Flüssigkeit zurück reflektiert. Da bei Resonanz die reflektierte Welle gleichphasig mit der entgegenkommenden, von der Ultraschallquelle 3 erzeugten Welle ist, findet eine Summierung ihrer Druckamplituden statt. Die in der Flüssigkeit verteilte Energie ist nach Gorelik G.S. "Kolebanija i volny-M.", F-ML, Moskau, 1959, 572 Seiten, proportional zum Quadrat der Druckamplitude. Das heißt, die Energieverluste der die Kavitation im Reaktor erzeugenden Schallwellen sind kleiner als die Energieverluste beim Stand der Technik.

Da die der Flüssigkeit zugewandten Oberflächen der Ultraschallquelle 3 und des Resonators 4 gleich groß sind, gleichen sich im in der Kammer 1 herrschenden Zustand die Schwingungsamplituden der Oberflächen aus. Sind die Oberflächen unterschiedlich groß ausgeführt, so sind die Schwingungsamplituden nach U.Mezona, ,,Fyzizeskaja akustika. Melody i pribory ultrazvukovych issledovanij" // pod red. U. Mezona.-M: Verlag MIR, Moskau, 1967, Band 1, Teil B, 362 Seiten, umgekehrt proportional dem Größenverhältnis der Oberflächen. Durch den Ausgleich wird einer Entstehung von Kavitationsgebieten im Bereich dieser Oberflächen entgegengewirkt bzw. die Entstehung von Kavitation unmittelbar an den Oberflächen verhindert, wodurch eine Erosion der Oberflächen der Ultraschallquelle 3 und des Resonators 4 verhindert wird.

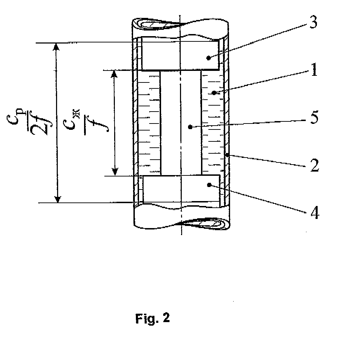

In Fig. 2 ist schematisch ein Detail des Kavitationsreaktors für den Fall dargestellt, dass die Ultraschallquelle 3 und der Resonator 4 auf solche Weise verbunden sind, dass sie ein einheitliches Festkörperresonanzschwingungssystem bilden.

Dieser Kavitationsreaktor weist eine zylindrische Kammer 1 für zu behandelnde Flüssigkeit auf. Die den Innenraum der Kammer 1 begrenzenden Wandungen sind Innenflächen eines Gehäuses 2, Oberfläche einer Ultraschallquelle 3 und die Oberfläche einer Reflexionswand. Die Ultraschallquelle 3 und der Resonator 4 sind untereinander mittels des Zylinders 5 verbunden. Zusammen bilden sie ein sogenanntes Monolitfestkörperschwingungssystem. Hierdurch wird sichergestellt, dass die Schwingungsamplituden der Oberflächen der Ultraschallquelle 3 und des Resonators 4 identisch sind. Die Bedingungen der Resonanz und der stehenden Welle werden dadurch sowohl in der Flüssigkeit als auch im Resonator eingehalten.

Auf der Basis des Festkörperschwingungssystems, das sowohl die Ultraschallquelle 3, als auch den Resonator 4 umfasst, kann eine Einrichtung verwirklicht werden, die mehrere Kavitationsreaktoren umfasst, die jeweils entsprechend dem Ausführungsbeispiel in Fig. 2 ausgeführt sind.

### Gewerbliche Anwendbarkeit

Die Erfindung kann in der Lebensmittel-, Chemie-, Pharma- und Parfümindustrie, in der Petrochemie, im Erdöl- und Erzbergbau sowie in der Medizin verwendet werden.

## Patentansprüche

1. Kavitationsreaktor zur Behandlung von Flüssigkeiten, mit einer füllbaren Kammer zur Bearbeitung von Flüssigkeiten, deren innerer Raum von den Oberflächen des Reaktorgehäuses begrenzt ist, mit mindestens einer Schallquelle akustischer Wellen und mindestens einer Reflexionswand,
ist **dadurch gekennzeichnet,**
**dass** er gemäß der Erfindung einen Festkörperresonator mit einer Resonanzfrequenz ausgestattet ist, die der Frequenz der Schallquelle gleich ist, die Reflexionswand ist dabei die Oberfläche des Resonators, die nach innen der Kammer gewendet ist.

2. Kavitationsreaktor gemäß Anspruch 1
**dadurch** ausgezeichnet,
dass einander zugewandte Oberflächen der Schallquelle und des Resonators im Bauch der Schwingungsversetzungen der zu bearbeitenden Flüssigkeit in der akustischen Welle untergebracht sind.

3. Kavitationsreaktor nach Anspruch 1 oder 2,
ist **dadurch** ausgezeichnet,
dass Schallquelle und der Resonator auf solche Weise verbunden sind, dass sie ein einheitliches Festkörperschwingungssystem bilden.
